# EUROPEAN PATENT SPECIFICATION

(11) **EP 1 166 795 B1**
(45) Date of publication and mention of the grant of the patent: **24.03.2004**
(21) Application number: 01113730.4
(22) Date of filing: 05.06.2001
(51) Int. Cl.: A61K 38/17, A61P 35/00

(54) **Skin cancer preventive agent containing sericin**
Sericin enthaltendes Mittel gegen Hautkrebs
Médicament contre le cancer de la peau contenant de la séricine

(30) Priority: 14.06.2000 JP 2000178776
(43) Date of publication of application: 02.01.2002
(73) Proprietor: Kabushiki Kaisha Aioi Hakko, Nishio-shi, Aichi-ken (JP); Seiren Kabushiki Kaisha, Fukui-shi, Fukui-ken (JP)
(72) Inventor: Jin, Zongxuan, Nishio-shi, Aichi-ken (JP); Muramatsu, Koichiro, Nishio-shi, Aichi-ken (JP); Yamada, Hideyuki, Fukui-shi, Fukui-ken (JP); Fuwa, Naozumi, Fukui-shi, Fukui-ken (JP); Hibasami, Hiroshige, Kawage-chom Age-gun, Mie-ken (JP)
(74) Representative: Leson, Thomas Johannes Alois, Dipl.-Ing.

(56) References cited:
- WO-A-00/51627
- PATENT ABSTRACTS OF JAPAN vol. 1998, no. 14, 31 December 1998 (1998-12-31) & JP 10 245345 A (SEIREN CO LTD), 14 September 1998 (1998-09-14)

## Description

### Background of the Invention

### 1. Field of the Invention

The present invention relates to a skin cancer preventive agent that uses natural sericin for its raw material, has high safety and stability, is free of adverse side effects, and can be used in fields such as drugs, over-the-counter drugs, health foods, cosmetics and lotions.

### 2. Description of the Related Art

Cancer is the leading cause of death, and it is predicted that the number of persons with cancer will continue to increase in the future. Thus, the discovery of a preventive method for preventing cancer is an extremely important subject. Although a diverse range of methods have been proposed for preventing cancer, chemoprophylaxis of cancer has recently attracted attention.

Chemoprophylaxis of cancer involves primary prevention of cancer that attempts to prevent the initiation and promotion of cancer by application of chemical substances. Those chemical substances for which effects have been confirmed in animal experiments or those that have been found to be effective based on epidemiological results are currently being tested in human subjects. Although chemical substances that prevent cancer are detected from animal experiments, research on chemoprophylaxis using animals is conducted using chemical carcinogenesis models. Although numerous stages of gene mutations are involved in the onset of cancer, carcinogenesis is a two-stage process consisting of initiation and promotion. Thus, chemoprophylactic agents for cancer consist of those that inhibit initiation of carcinogenesis by the application of a cancer chemoprophylactic agent during the initiation stage of an animal chemical carcinogenesis model, and those inhibit promotion of carcinogenesis by application during the promotion stage. Furthermore, cancer preventive agents are normally required to be free of problems with safety and not have any adverse side effects, while also being able to be applied over a long period of time.

In consideration of the above requirements, the object of the present invention is to provide a skin cancer preventive agent that has high levels of safety and stability while being free of adverse side effects.

The documents WO-A-0051627 and JP-A-10254354 disclose compositions containing sericin, which could be used for the prevention of colon cancer (former document) or for the prevention of dermatitis (latter document), respectively.

### Summary of the Invention

As a result of focusing on the naturally-occurring cocoon protein, sericin, the inventors of the present invention found that sericin exhibits an inhibitory action on promotion of skin carcinogenesis at low concentrations, thereby leading to completion of the present invention. Namely, the invention of claim 1 relates to the use of sericin for the manufacture of a medicament effective in the prevention of skin cancer.

The invention of claim 2 relates to the use according to claim 1 wherein, the sericin is a hydrolysis product of sericin.

In addition, the invention of claim 3 relates to the use according to either of claims 1 or 2 wherein, the weight average molecular weight of sericin is from 5,000 to 100,000.

### Brief Description of the Drawings

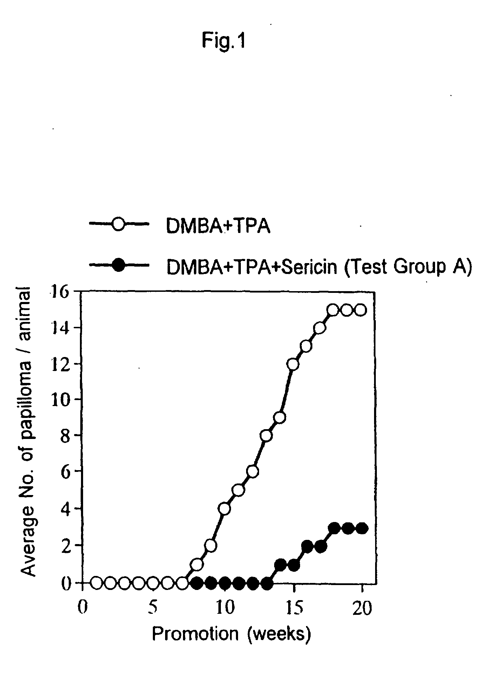

Fig. 1 is a graph indicating the time-dependent inhibitory action on the number of skin papilloma in mice according to one example of the present invention.

### Description of the Preferred Embodiment

The present invention is directed to the use of a naturally-occurring preventive agent for skin cancer having sericin (including hydrolysis products of sericin) for its active ingredient. The sericin used in the present invention is normally extracted by solvent or physically separated from the cocoon or raw silk expelled by silkworms.

Domesticated silkworms raised by breeders or wild silkworms thriving in the natural environment can be used for the silkworms used in the present invention, and are not limited to any particular species. The cocoon may contain a pupa, a portion of the cocoon may be cut open and the pupa removed, or the cocoon may be crushed. The raw silk may be reeled, woven, sewn or crushed.

Examples of extraction solvents for obtaining sericin from the above materials include cold water, hot water, water-containing alcohol and other hydrophilic solvents. For example, after boiling domesticated silkworm cocoons for several minutes in 20 to 30 volumes of hot water, the solid portion is removed by filtration, centrifugation and so forth. There are no particular restrictions on the methods for extraction, and any suitable methods may be used including those known in the prior art.

Moreover, sericin having a weight average molecular weight from 5,000 to 100,000 and a particularly superior inhibitory action on promotion of skin carcinogenesis can be obtained by isolating and purifying the resulting extract. If the weight average molecular weight is less than 5,000, the inhibitory action on promotion of skin carcinogenesis decreases, and in the case the weight average molecular weight exceeds 100,000, mixing with other suitably used drugs such as carriers, fillers and diluents becomes poor. Consequently, sericin (and including hydrolysis products of sericin) having a weight average molecular weight from 5,000 to 100,000 are used preferably.

There are no particular restrictions on the isolation and purification methods used here. For example, known methods such as salting out, organic solvent precipitation, gel filtration chromatography, ion exchange chromatography, reverse phase chromatography, reverse osmosis, ultrafiltration, ultracentrifugation and electrolysis can be used either alone or in combination. Moreover, drying may be carried out by freeze-drying, spray drying and so forth.

Although the sericin having superior inhibitory action on promotion of skin carcinogenesis obtained in this manner has a high molecular weight, it is highly hydrophilic, dissolves easily in cold water, has good miscibility with other drugs, and its aqueous solutions do not form gel, have low viscosity and can be handled easily.

The skin carcinogenesis preventive agent used in the present invention can be applied orally, intraperitoneally or intravenously either directly or in the form of a powder, granules, tablets, capsules, injection, gel, stick or solution and so forth by mixing with a suitable carrier, filler or diluent and so forth. In addition, it can also be used as a skin external preparation. Although there are no particular restrictions on the form of the skin external preparation, examples include creams, milky lotions, packs, lotions, powder oils and ointments, and can be used by blending with a base comprised of normally used raw materials of cosmetics, pharmaceuticals and so forth. The required amount can also be ingested in the form of a food by mixing with food.

Since sericin is a naturally-occurring substance, it is highly safe, and can be added to drugs, over-the-counter drugs, cosmetics, foods and health foods. Consequently, it can be ingested easily during the course of daily life. The amount added is normally 0.1 to 50 wt%, and preferably 0.5 to 5.0 wt%. Although adequate effects are demonstrated even if only a small amount is added, since it has no toxicity and excellent water solubility, no noteworthy problems are encountered even if added or ingested in large amounts.

The following provides an explanation of specific examples of the present invention.

100 g of cocoons were added to 500 ml of boiled distilled water and boiled for 1 hour. After cooling by allowing to stand at room temperature, the cocoons were filtered out to obtain an extract. This extract was applied to gel filtration chromatography to obtain a fraction having a molecular weight from 10,000 to 50,000 followed by freeze-drying to obtain solid sericin having a weight average molecular weight of 30,000. Moreover, a solution in which this solid sericin was dissolved in distilled water to a concentration of 2.5% (first example of the present invention) and a solution in which this solid sericin was dissolved in distilled water to a concentration of 5% (second example of the present invention) were additionally obtained.

The following experiment was conducted to investigate the inhibitory action on promotion of skin carcinogenesis using the sericin solutions of the above first and second examples obtained in the manner described above.

### (Experiment)

Eight-week-old male DDY mice were divided into groups of 5 animals each. These were designated as the control group, test group A and test group B, respectively. The animals were housed at a room temperature of 23°C using a light-dark cycle of 12 hours while providing food and water. After shaving the backs of the animals of the control group, the skin carcinogenesis initiator, 7,12-dimethylbenzen[ α ]anthracene (DMBA), was dissolved in acetone, and 0.2 µmol of DMBA per animal were applied to the skin on the backs of the above mice. Two weeks later, the carcinogenesis promoter, 12- *o* -tetradecanoyl-phorbol-13-acetate (TPA), was dissolved in acetone and 10 nmol of TPA per animal were applied at the same site on the skin of the mice three times a week for 20 weeks to induce skin cancer in the mice.(Control group)

In parallel with the above experiment using the control group, DMBA and TPA were applied in the same manner as the control group to the mice of test groups A and B, and for each application of TPA, the 2.5% sericin solution of the first example was applied to the skin of the mice of test group A, while the 5% sericin solution of the second example was applied to the skin of the mice of test group B, after each application of TPA. A total of 100 µl of each solution was applied three times a week for 20 weeks. The total amount of sericin applied was 2.5 mg in the mice of test group A, and 5 mg in the mice of test group B.

The time-dependent inhibitory action on skin carcinogenesis in mice by the present invention (sericin) with respect to promotion of skin carcinogenesis was investigated for the above test group A as compared with the control group. The carcinogenesis inhibition rate of mouse skin cancer was determined by determining the number of papilloma having a diameter of 2 mm or more that formed on the skin of the mice. As is clear from the results show in Fig. 1, although initial papilloma occurred in week 8 in the control group to which only acetone solutions of DMBA and TPA were applied, in test group A to which the sericin of the first example was applied, initial papilloma did not occur until week 14. In addition, as a result of comparing the number of papilloma in week 20, the occurrence of papilloma was found to be inhibited by 80% in test group A as compared with the control group. In this manner, the skin cancer preventive agent of the first example was clearly determined to exhibit preventive effects on the occurrence of papilloma on mice skin.

In addition, when the numbers of papilloma were determined in the groups of the above first and second examples in week 20 in order to investigate the concentration-dependent inhibitory action of sericin on the number of papilloma on mice skin, the number of papilloma was lower in test group B, in which the total amount applied was 5 mg, than in test group A, in which the total amount applied was 2.5 mg. In addition, when test group B was compared with the control group in week 20, an inhibition rate of 100% was demonstrated in test group B. On the basis of these findings, it was determined that the larger the sericin concentration (applied amount), the greater the preventive effect on the occurrence of mouse skin papilloma.

On the basis of these results, it was clearly determined that application of the skin cancer preventive agent used in the present invention makes it possible to confirm that the promotion of carcinogenesis of mouse skin can be inhibited, and that the skin cancer preventive agent used in the present invention has a function that prevents the occurrence of skin cancer.

As has been explained above, according to the skin cancer preventive agent used in the present invention, the use of sericin makes it possible to inhibit the occurrence of skin cancer with high levels of safety and stability without the occurrence of adverse side effects, while also allowing long-term use.

In addition, the skin cancer preventive agent used in the present invention is a functional ingredient that prevents skin cancer in a wide range of fields, including drugs, skin external preparations, over-the-counter drugs, cosmetics and lotions.

The present invention provides the use of a skin cancer preventive agent for the manufacture of a medicament that inhibits the promotion of carcinogenesis of skin cancer while having high levels of safety and stability as well as being free of adverse side effects. The present invention is characterized by using sericin.

## Claims

1. Use of sericin for the manufacture of a medicament effective in the prevention of skin cancer.

2. The use according to claim 1, wherein the sericin is a hydrolysis product of sericin.

3. The use according to either of claims 1 or 2, wherein the average molecular weight of sericin is from 5,000 to 100,000.

4. The use according to any of claims 1 to 3, wherein the sericin is added to drugs, over-the-counter drugs, cosmetics, lotions, foods or health foods.

5. The use according to any of claims 1 to 3, wherein the form of the medicament is selected from the group consisting of a powder, granules, tablets, capsules, injection, gel, stick, solution, creams, milky lotions, lotions, packs, powder oils and ointments.

## Patentansprüche

1. Verwendung von Sericin für die Herstellung eines in der Hautkrebsprävention wirksamen Medikaments.

2. Verwendung nach Anspruch 1, wobei das Sericin ein Hydrolyseprodukt von Sericin ist.

3. Verwendung nach einem der Ansprüche 1 oder 2, wobei die massegemittelte Molekülmasse von Sericin von 5.000 bis 100.000 ist.

4. Verwendung nach einem der Ansprüche 1 bis 3, wobei das Sericin zu Arzneimitteln, rezeptfreien Arzneimitteln, Kosmetika, Lotionen, Lebensmitteln oder Gesundheitslebensmitteln zugegeben wird.

5. Verwendung nach einem der Ansprüche 1 bis 3, wobei die Form des Medikaments ausgewählt ist aus der Gruppe bestehend aus einem Pulver, Körnern, Tabletten, Kapseln, Injektionen, Gel, Stift, Lösungen, Creme, milchigen Lotionen, Lotionen, Packungen, Pulverölen und Salben.

## Revendications

1. Utilisation de la séricine pour la fabrication d'un médicament efficace dans la prévention du cancer de la peau.

2. Utilisation selon la revendication 1, dans laquelle la séricine est un produit de l'hydrolyse de la séricine.

3. Utilisation selon l'une quelconque des revendications 1 ou 2, dans laquelle le poids moléculaire moyen de la séricine est compris entre 5 000 et 100 000.

4. Utilisation selon l'une quelconque des revendications 1 à 3, dans laquelle la séricine est ajoutée à des médicaments, à des médicaments en vente libre, à des produits de beauté, à des lotions, à des aliments ou à des produits diététiques.

5. Utilisation selon l'une quelconque des revendications 1 à 3, dans laquelle la forme médicamenteuse est choisie dans le groupe comprenant les poudres, granules, comprimés, gélules, injections, gels, sticks, solutions, crèmes, laits, lotions, enveloppements, huiles poudreuses et pommades..
